(19) Europäisches Patentamt
European Patent Office
Office européen des brevets

(11) Veröffentlichungsnummer: **0 225 543**
**B1**

(12) # EUROPÄISCHE PATENTSCHRIFT

(45) Veröffentlichungstag der Patentschrift: **27.09.89**

(51) Int. Cl.⁴: **C07C 95/08**, A61K 31/135

(21) Anmeldenummer: **86116409.3**

(22) Anmeldetag: **26.11.86**

(54) **Basisch substituierte Phenylacetaldehyde, ihre Herstellung und diese enthaltende Arzneimittel.**

(30) Priorität: **05.12.85 DE 3542994**

(43) Veröffentlichungstag der Anmeldung: **16.06.87 Patentblatt 87/25**

(45) Bekanntmachung des Hinweises auf die Patenterteilung: **27.09.89 Patentblatt 89/39**

(84) Benannte Vertragsstaaten: **AT BE CH DE ES FR GB IT LI NL SE**

(56) Entgegenhaltungen:
EP-A- 0 064 158
DE-C- 924 862
DE-C- 1 154 810
FR-A- 1 469 468

(73) Patentinhaber: **BASF Aktiengesellschaft, Carl-Bosch-Strasse 38, D-6700 Ludwigshafen(DE)**

(72) Erfinder: **Seitz, Werner, Dr., Bismarckstrasse 226, D-6831 Plankstadt(DE)**
Erfinder: **Baldinger, Verena, Dr., Schiffsgasse 6, D-6900 Heidelberg(DE)**
Erfinder: **Gries, Josef, Dr., Roemerweg 43, D-6706 Wachenheim(DE)**
Erfinder: **Lenke, Dieter, Prof. Dr., Kekuleplatz 1, D-6700 Ludwigshafen(DE)**
Erfinder: **Raschack, Manfred, Dr., Donnersbergstrasse 7, D-6714 Weisenheim am Sand(DE)**
Erfinder: **Ruebsamen, Klaus, Dr., Erschigweg 19, D-6730 Neustadt(DE)**

EP 0 225 543 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue basisch substituierte Phenylacetaldehyde, deren Herstellung sowie Arzneimittel, welche diese Substanzen enthalten.

In der DE-PS 1 154 810 bzw. EP-OS 64 158 sind basisch substituierte Phenylacetonitrile beschrieben. Aus dieser Verbindungsklasse haben sich in der Therapie der koronaren Herzkrankheiten und des Bluthochdrucks das Verapamil und das Gallopamil bewährt. Für das Verapamilmolekül sind Zusammenhänge zwischen chemischer Struktur und biologischer Wirkung verschiedentlich publiziert worden (vgl. Arzneim. Forsch./Drug Res. 5 (1981), 773). Aufgrund dieser Struktur-Wirkungs-Betrachtungen und experimenteller Arbeiten wurde von Mannhold [Drugs of Today 20 (2), 69-90 (1984)] nachgewiesen, daß die Nitrilgruppe des Verapamilmoleküls essentiell für die biologische Wirkung ist.

Es wurden nun Verbindungen gefunden, die trotz Abwandlung der Nitrilgruppe hochwirksam sind.

Die Erfindung betrifft neue basisch substituierte Phenylacetaldehyde der Formel I

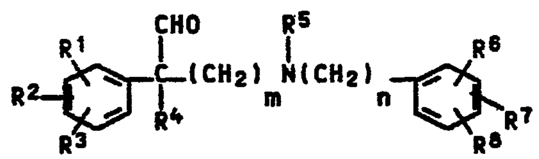

I,

worin

$R^1$, $R^2$, $R^3$, $R^6$, $R^7$, $R^8$ gleich oder verschieden sind und Wasserstoffatome, Halogenatome, Trifluormethylgruppen, $C_1$-$C_4$-Alkylgruppen, Nitrogruppen oder $C_1$-$C_4$-Alkoxygruppen bedeuten, wobei auch jeweils zwei Reste in Nachbarstellung zusammen Methylendioxy-, Ethylendioxy-, 1,3-Dioxatetramethylen-, Propylen- oder Butylengruppen bilden können,

$R^4$ eine gesättigte oder ungesättigte $C_1$-$C_{12}$-Alkylgruppe, eine Cycloalkyl- oder Arylgruppe ist,

$R^5$ einen $C_1$-$C_4$-Alkylrest darstellt und

m und n gleich oder verschieden sind und die Zahlen 2 bis 4 bedeuten, sowie deren Salze mit physiologisch verträglichen Säuren.

Als Halogenatome für $R^1$ bis $R^3$ und $R^6$ bis $R^8$ kommen bevorzugt Fluor- und Chloratome in Betracht. Bevorzugte Alkyl- und Alkoxygruppen für $R^1$ bis $R^3$ und $R^5$ bis $R^8$ sind solche mit 1 bis 2 Kohlenstoffatomen. Bevorzugte Nitroverbindungen sind solche mit einer Nitrogruppe.

Besonders interessant sind die folgenden Verbindungen:

(RS)-2-[3-[(Phenethyl)methylamino]propyl]-2-isopropyl-phenylacetaldehyd,
(R)-2-[3-[(Phenethyl)methylamino]propyl]-2-isopropyl-phenylacetaldehyd,
(S)-2-[3-[(Phenethyl)methylamino]propyl]-2-isopropyl-phenylacetaldehyd,
(RS)-2-[3-[(3-Methoxyphenethyl)methylamino]propyl]-2-isopropyl-3,4-dimethoxyphenylacetaldehyd,
(R)-2-[3-[(3-Methoxyphenethyl)methylamino]propyl]-2-isopropyl-3,4-dimethoxyphenylacetaldehyd,
(S)-2-[3-[(3-Methoxyphenethyl)methylamino]propyl]-2-isopropyl-3,4-dimethoxyphenylacetaldehyd,
(RS)-2-[3-[(3-Methoxyphenethyl)methylamino]propyl]-2-isopropyl-3,4,5-trimethoxyphenylacetaldehyd,
(R)-2-[3-[(3-Methoxyphenethyl)methylamino]propyl]-2-isopropyl-3,4,5-trimethoxyphenylacetaldehyd,
(S)-2-[3-[(3-Methoxyphenethyl)methylamino]propyl]-2-isopropyl-3,4,5-trimethoxyphenylacetaldehyd,
(RS)-2-[3-[(3,5-Dimethoxyphenethyl)methylamino]propyl]-2-isopropyl-3,5-dimethoxyphenylacetaldehyd,
(R)-2-[3-[(3,5-Dimethoxyphenethyl)methylamino]propyl]-2-isopropyl-3,5-dimethoxyphenylacetaldehyd,
(S)-2-[3-[(3,5-Dimethoxyphenethyl)methylamino]propyl]-2-isopropyl-3,5-dimethoxyphenylacetaldehyd,
(RS)-2-[3-[(3-Ethoxyphenethyl)methylamino]propyl]-2-isopropyl-3-ethoxyphenylacetaldehyd,
(R)-2-[3-[(3-Ethoxyphenethyl)methylamino]propyl]-2-isopropyl-3-ethoxyphenylacetaldehyd,
(S)-2-[3-[(3-Ethoxyphenethyl)methylamino]propyl]-2-isopropyl-3-ethoxyphenylacetaldehyd,
(RS)-2-[3-[(3,5-Diethoxyphenethyl)methylamino]propyl]-2-isopropyl-3,5-diethoxyphenylacetaldehyd,
(R)-2-[3-[(3,5-Diethoxyphenethyl)methylamino]propyl]-2-isopropyl-3,5-diethoxyphenylacetaldehyd,
(S)-2-[3-[(3,5-Diethoxyphenethyl)methylamino]propyl]-2-isopropyl-3,5-diethoxyphenylacetaldehyd.

Als physiologisch verträgliche Säuren kommen z.B. in Frage: Salzsäure, Bromwasserstoffsäure, Schwefelsäure, Phosphorsäure, Essigsäure, Maleinsäure, Milchsäure, Weinsäure, Zitronensäure und Fumarsäure.

Die neuen Verbindungen besitzen mindestens ein asymmetrisches C-Atom und liegen daher in den verschiedenen enantiomeren Formen vor. Folglich können die Verbindungen I entweder in optisch aktiven Formen oder als racemische Mischungen hergestellt werden. Die Racemate der Verbindungen I können durch herkömmliche Techniken, z.B. durch Trennung (fraktionierte Kristallisation, Säulenchromatographie) der diastereomeren Salze, in ihre optische Antipoden gespalten werden. Die diastereomeren Salze sind durch Umsetzung der Verbindungen I mit chiralen Säuren herstellbar. Die enantiomeren Formen können auch durch die Verwendung optisch aktiver Ausgangsverbindungen erhalten werden.

Die neuen Verbindungen werden hergestellt, indem man
a) Phenylacetonitrile der Formel II

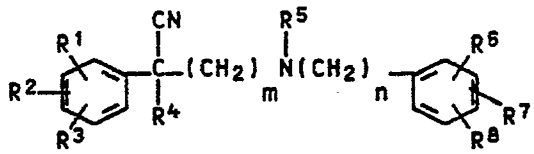

II,

worin $R^1$ bis $R^8$, m und n wie oben angegeben definiert sind, mit einem komplexen Aluminiumhydrid umsetzt und anschließend hydrolisiert oder
b) Phenylacetaldehyde der Formel III

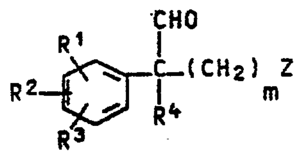

III,

worin $R^1$ bis $R^4$ und m die oben angegebenen Bedeutungen haben und Z eine Austrittsgruppe darstellt, mit einem Phenylalkylamin der Formel IV

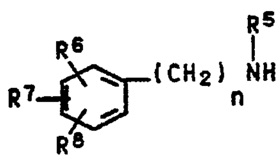

IV,

worin $R^5$ bis $R^8$ und n die obige Bedeutung besitzen, zur Reaktion bringt und die so erhaltene Verbindung gewünschtenfalls in ein Salz mit einer physiologisch verträglichen Säure überführt.

Folgende komplexe Aluminiumhydride für die Umsetzung a) kommen u.a. in Frage: Lithiumaluminiumhydrid, Natrium-bis-(2-methoxyethoxy)-aluminiumdihydrid, Lithium- bzw. Natrium-triethoxyaluminiumhydrid und bevorzugt Diisobutylaluminiumhydrid.

Die Umsetzung wird bevorzugt in einem aprotischen Lösungsmittel, wie Toluol oder Hexan, in aliphatischen und cyclischen Ethern, wie Diethylether oder Tetrahydrofuran, durchgeführt. Die Reaktionstemperatur kann von -60°C bis +20°C gewählt werden. Bevorzugt wird die Reaktion zwischen -20°C und 0°C durchgeführt.

Die Umsetzung b) wird bevorzugt in einem dipolar aprotischen Lösungsmittel, wie z.B. Acetonitril, Dimethylformamid, Hexamethylphosphorsäuretriamid, unter Zusatz eines säurebindenden Mittels, z.B. wasserfreies Kaliumcarbonat, durchgeführt. Als säurebindendes Mittel kann auch ein zusätzliches Äquivalent des Phenylalkylamins der Formel IV eingesetzt werden. Die Reaktionstemperatur kann von Raumtemperatur bis 120°C gewählt werden. Bevorzugt wird die Reaktion zwischen 70°C und 100°C durchgeführt.

Als Austrittsgruppen eignen sich beispielsweise Chlor, Brom oder Jod, Schwefelsäureester, Mesylate oder Triflate.

Phenylacetaldehyde der Formel III können durch Reduktion der entsprechenden Phenylacetonitrile mit komplexen Aluminiumhydriden erhalten werden.

Die Verbindungen der allgemeinen Formel I bzw. deren physiologisch verträgliche Säureadditionssalze haben wertvolle pharmakologische Eigenschaften. Sie wirken als hochaktive Ca-Antagonisten dilatierend auf periphere und zentrale Gefäße und schützen Herz und Gefäße vor Schäden die durch gesteigerte Ca-Mobilisierung bzw. Ca-Überladung hervorgerufen werden. Die Verbindungen hemmen die Magensäuresekretion und besitzen cytoprotektive und antiulceröse Wirkung. Schließlich sind sie in der Lage, Spasmen der Bronchialmuskulatur zu verhindern oder zu lösen.

Das zeigen folgende Versuche:

Hemmung der Magensäuresekretion

Die Hemmung der Magensäuresekretion äußert sich in einem Anstieg des pH-Wertes der Magenschleimhautoberfläche. Für diese Messung wurden Gruppen von 5 bis 10 weiblichen Sprague-Dawley-Ratten (160 bis 190 g), die 48 Stunden ohne Futter waren (Wasser ad libitum), mit unterschiedlichen Dosen der Prüfsubstanzen (p.o.) vorbehandelt. 1 Stunde später wurde unter Halothan-Inhalationsnarkose eine pH-Sonde (Ingold 440 $M_3$) in den Magen eingeführt und der pH-Wert auf der Schleimhautoberfläche gemessen (pH-Wert bei unbehandelten Tieren:
1.40 ± 0.02, N = 200). Aus der linearen Regression zwischen log Dosis und der Erhöhung des pH-Wertes wird als ED 0,75 die Dosis bestimmt, die eine Zunahme des pH-Wertes um 0,75 pH-Einheiten bewirkt.

Cytoprotektive Wirkung

Die cytoprotektive Wirkung wurde an Gruppen von 8 weiblichen Sprague-Dawley-Ratten (160 bis 190 g) bestimmt, die für 48 Stunden kein Futter erhielten (Wasser ad libitum) und mit unterschiedlichen Dosen der Prüfsubstanzen (p.o.) vorbehandelt wurden. 1 Stunde später wurde den Tieren 10 ml/kg 100 % Ethanol per os verabreicht. 1 Stunde später wurden die Tiere durch $CO_2$-Inhalation getötet, die Mägen entnommen und die Intensität der Läsionen nach folgendem Schema beurteilt:
0 = unveränderte Schleimhaut
1 = wenig schmale, längliche oder kleine flächige Rötungen
2 = breite, längliche oder großflächige unterbrochene Rötungen
3 = starke Rötungen, die fast die gesamte Schleimhaut des Drüsenmagens bedecken.

Aus der linearen Regression zwischen log Dosis und der Verringerung der Intensität der Läsionen der Magenschleimhaut wird als ED 50 % die Dosis bestimmt, die die Läsionen um 50 % verringert.

Antiulceröse Wirkung

Zur Untersuchung der antiulcerösen Wirkung erhielten Gruppen von 10 weiblichen Sprague-Dawley-Ratten (160 bis 180 g) 1 mg/kg Reserpin i.p. und blieben danach 18 Stunden ohne Futter (Wasser ad libitum). Nach dieser Zeit erhielten die Tiere 21,5 mg/kg Indomethacin i.p. und die Prüfsubstanz per os. Anschließend wurden sie 6 Stunden lang bei einer Temperatur von 8°C gehalten und danach getötet. Die Mägen wurden entnommen und die Fläche der ulcerösen Schleimhautläsionen bestimmt. Aus den linearen Regressionen zwischen den Logarithmen der applizierten Dosen und der relativen Verkleinerung der Fläche der Ulcerationen bezogen auf die Kontrolltiere wird als ED 50 % die Dosis bestimmt, die eine Verkleinerung der Ulcusfläche um 50 % bewirkt.

**Tabelle**

**Magensäuresekretionshemmende, cytoprotektive und antiulceröse Wirkungen an der Ratte, Appl. per os**

| Beispiel-Nr. | Säuresekretionshemmende Wirkung ED 0,75[1)] | Cytoprotektive Wirkung ED 50% | Antiulceröse Wirkung ED 50% |
|---|---|---|---|
| Verapamil | 10,5 | 2) | 2) |
| 3 | 2,1 | 8,3 | 3,8 |
| 2 | 2,0 | 9,4 | 12,2 |

1) Dosen in mg/kg
2) keine Wirkung bis 46,4 mg/kg

Die erfindungsgemäßen Verbindungen bewirken, wie an Beispiel 3 und 2 nachgewiesen wurde, in fünfmal niedrigeren Dosen als die Vergleichssubstanz Verapamil eine Hemmung der Magensäuresekretion.

Sie schützen darüber hinaus die Magenschleimhaut vor dem schädigenden Einfluß von Ethanol und verhindern die Ausbildung von Magenulcera.

Die cytoprotektive und die antiulceröse Wirkung sind zwei zusätzliche Wirkkomponenten, die bei Verapamil nicht nachzuweisen sind.

Blutdrucksenkende und antihypertensive Wirkung

Zur Feststellung der blutdrucksenkenden Wirkung wurden Sprague-Dawley-Ratten (230 bis 280 g) mit Urethan (1,78 g/kg i.p.) narkotisiert. Der Blutdruck wurde in der A.carotis gemessen. Die Substanzen wurden i.v. in die V.jugularis appliziert. Als ED 20 % wird aus der linearen Regression zwischen log Dosis (mg/kg) und relativer Blutdrucksenkung (Δ %) die Dosis berechnet, die eine 20 %ige Blutdrucksenkung bewirkt.

Zur Feststellung der antihypertensiven Wirkung wurden die Substanzen männlichen spontan hypertonen Okamoto-Ratten (4 bis 8 Tiere/Dosis, Gewicht 270 bis 360 g) oral appliziert. Der systolische Blutdruck wurde vor und 2 Stunden nach der Applikation unblutig am Rattenschwanz mit Hilfe von Piezokristallaufnehmern ermittelt. Als ED 20 % wird unter Berücksichtigung der Werte unbehandelter Kontrolltiere die Dosis bestimmt, welche den systolischen Druck um 20 % senkt.

| Tabelle | | | | |
|---|---|---|---|---|
| Blutdrucksenkende Wirkung | | | | |
| Beispiel-Nr. | Blutdrucksenkung Ratte, nark., ED 20% i.v. | | Blutdrucksenkung SH-Ratte ED 20% per os | |
| | mg/kg | R.W. | mg/kg | R.W. |
| Verapamil | 0,34 | 1,0 | 25 | 1,00 |
| 12 | 0,30 | 1,13 | 4,0 | 6,25 |

Wie aus der Tabelle hervorgeht, senkt die Substanz des Beispiels 12 bei i.v. Applikation an der narkotisierten Ratte den Blutdruck etwas stärker als Verapamil.

Besonders stark wirksam erweist sie sich bei oraler Applikation an wachen spontan hypertonen Ratten. In diesem Modell senkt die Substanz des Beispiels 12 den Blutdruck im Vergleich zu Verapamil in 6,3-fach kleinerer Dosierung.

Broncholytische Wirkung

Zur Prüfung der broncholytischen Wirkung wurden Meerschweinchen im Gewicht von 300 bis 450 g mit Urethan (1,5 g/kg, i.p.) sowie nach der Präparation mit Pentobarbital (25 mg/kg, i.v.) narkotisiert und die Trachea und die V.jugularis kanüliert.

Die Tiere wurden mit einer Starling-Pumpe künstlich beatmet. Durch Injektion von Histamin (0,001 bis 0,00464 mg/kg) oder Acetylcholin (0,02 bis 0,04 mg/kg), 2 bis 3-mal im Abstand von 10 min wurden Bronchospasmen ausgelöst.

Diese wurden über induktive Druckaufnehmner nach der Methode von Konzett und Rössler (1940) bestimmt.

Die Substanzen, die 5 min vor Auslösung der Bronchospasmen i.v. injiziert werden, hemmen diese dosisabhängig.

Für Vergleiche werden als ED 75 % aus den linearen Regressionen zwischen log Dosis und Spasmenhemmung die Dosen bestimmt, die eine 75 %ige Hemmung der Bronchospasmen bewirken.

| Tabelle | | |
|---|---|---|
| Broncholytische Wirkung am Meerschweinchen, Appl. i.v. | | |
| Beispiel-Nr. | ED 75%, mg/kg | |
| | Histaminbronchospasmus | Acetylcholinbronchospasmus |
| Verapamil | 1,3 | 0,56 |
| 8 | 0,38 | 0,23 |
| 6 | 0,59 | 0,39 |
| 7 | 0,61 | 0,67 |
| 12 | 0,65 | 0,27 |

Die Tabelle zeigt die broncholytische Aktivität der erfindungsgemäßen Substanzen und weist an den Substanzen der Beispiele 8, 6, 7 und 12 im Vergleich zur Referenzsubstanz Verapamil eine deutlich höhere Wirksamkeit aus.

Calciumantagonistische Wirkung an Aortenspiralstreifen

Die Versuche der Ca-antagonistischen Wirkung werden an Aortensprialstreifen männlicher und weiblicher Sprague-Dawley-Ratten im Körpergewicht von 200 bis 300 g durchgeführt.

Die Tiere werden mit Ether getötet und Thorax- sowie Brustaorta entnommen. Pro Tier werden maximal 6 Spiralstreifen von etwa 2 mm Breite und 2 cm Länge verwendet.

Die Aortenstreifen werden in eine modifizierte Tyrodelösung von 37°C eingehängt, initial mit 1,5 g vorgespannt und nach einer Relaxationszeit von ca. 1 h Ca-verarmt, indem sie 5 min in Ca-freier Tyrodelösung mit Zusatz von 0,2 mM Na-EDTA gehalten werden.

Die Ca-freien Gefäßstreifen werden mit K-reicher Tyrodelösung 10 min lang depolarisiert. Die Ca-Kontraktion wird durch eine $CaCl_2$-Konzentration von 0,5 mM hervorgerufen. Nach 15 min werden die Gefäßstreifen mit Ca-freier Tyrodelösung mit 0,2 mM NaEDTA wiederum Ca-verarmt.

Darauf wird erneut 10 min mit K-reicher Tyrodelösung depolarisiert und anschließend die Prüfsubstanz in 0,05 ml zugegeben. Nach 15 min Einwirkzeit wird durch erneute Zugabe von $CaCl_2$ in der Konzentration 0,5 mM geprüft, ob die Testsubstanz Ca-antagonistische Wirkung besitzt. Es wird angegeben, um wieviel Prozent der Effekt von 0,5 mM $Ca^{++}$ durch die antagonistische Substanz gehemmt wird. Als EC 50 % wird die Konzentration bestimmt (an mindestens 12 Gefäßstreifen) die die Hemmung des Ca-Effektes um 50 % bewirkt.

**Tabelle**

**Ca-antagonistische Wirkung an isolierten Aortenspiralstreifen der Ratte, Appl. in vitro**

| Beispiel-Nr. | EC 50%, mol/l |
|---|---|
| Verapamil | $3{,}5 \times 10^{-8}$ |
| 7 | $2{,}2 \times 10^{-8}$ |
| 12 | $3{,}2 \times 10^{-8}$ |
| 11 | $1{,}2 \times 10^{-8}$ |
| 10 | $1{,}9 \times 10^{-8}$ |
| 9 | $1{,}2 \times 10^{-9}$ |

Die Tabelle zeigt die Ca-antagonistische Aktivität der erfindungsgemäßen Substanzen und weist an den Subtanzen der Beispiele 11, 10 und 9 im Vergleich zur Referenzsubstanz Verapamil eine deutlich höhere Wirksamkeit aus.

Aufgrund dieser Wirkungen können die erfindungsgemäßen Verbindungen z.B. zur Prophylaxe und Behandlung der koronaren Herzkrankheit und als Antihypertensiva zur Behandlung des Bluthochdrucks, sowie bei peripheren und zentralen Durchblutungsstörungen und cerebralen Sauerstoff-Mangelerscheinungen eingesetzt werden.

Ferner können sie bei Magenerkrankungen, die mit Hypersekretion einhergehen, und zur Behandlung von Magen- und Duodenalulcera, sowie als Broncholytica bei bronchospastischen Zuständen Verwendung finden.

Die neuen Verbindungen können in üblicher Weise oral oder parenteral gegeben werden. Die Dosierung hängt vom Alter, Zustand und Gewicht des Patienten sowie von der Applikationsart ab. In der Regel beträgt die tägliche Wirkstoffdosis zwischen etwa 0,1 und 10 mg/kg Körpergewicht bei oraler Gabe und zwischen 0,01 und 1,0 mg/kg Körpergewicht bei parenteraler Gabe. Im Normalfall werden tägliche Dosen von 1 bis 5 mg/kg oral und 0,05 bis 0,25 mg/kg parenteral angewendet.

Die erfindungsgemäßen Wirkstoffe können in die üblichen galenischen Anwendungsformen, wie Tabletten, Dragees, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffektes, wie Carboxipolymethylen, Carboximethylcellulose, Celluloseacetatphthalat oder Polyvinylacetat erhalten werden.

Die Tabletten können auch aus mehreren Schichten bestehen. Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmacksverbesserndes Mittel, z.B. Aromastoffe, wie Vanillin oder Orangenextrakt, enthalten.

Sie können außerdem Suspendierhilfsstoffe oder Dickungsmittel, wie Natriumcarboximethylcellulose, Netzmittel, beispielsweise Kondensationsprodukte von Fettalkoholen mit Ethylenoxid oder Schutzstoffe, wie p-Hydroxibenzoate, enthalten.

Injektionslösungen werden in üblicher Weise, z.B. unter Zusatz von Konservierungsmitteln, wie p-Hydroxibenzoaten oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyethylenglykol bzw. dessen Derivaten, herstellen.

Die erfindungsgemäßen Verbindungen sind auch für die Kombination mit anderen pharmakodynamisch wirksamen Stoffen, wie Diuretika, Thrombozytenaggregationshemmern, geeignet.

Die folgenden Beispiele erläutern die Erfindung, ohne sie zu beschränken:

Beispiel 1

2-[3-[(Phenethyl)methylamino]propyl]-2-isopropyl-phenylacetaldehyd

Zu einer Lösung von 33,4 g (0,1 Mol) 2-[3-[(Phenethyl)methylamino]propyl]-2-isopropyl-phenylacetonitril in 400 ml Diethylether tropfte man bei -5°C 180 ml einer 1 m Lösung von Diisobutylaluminiumhydrid in Hexan. Das Gemisch wurde 1,5 h nachgerührt und anschließend mit 500 ml 10%iger Schwefelsäure versetzt. Nach Zugabe von 30 g Weinsäure wurde mit konzentrierter Kalilauge alkalisch gestellt, die Etherphase abgetrennt und mehrmals mit Kochsalzlösung gewaschen. Nach Trocknen über Natriumsulfat destillierte man den Ether ab, löste den verbleibenden öligen Rückstand in 300 ml Ethylacetat und versetzte mit isopropanolischer Salzsäure. Nach Stehen über Nacht wurde das ausgefallene Hydrochlorid abgesaugt.
Ausbeute: 31,5 g (85 %) Hydrochlorid
Fp 166 - 168°C

Analog wurden erhalten:

Beispiel 2

(S)-2-[3-[(Phenethyl)methylamino]propyl]-2-isopropyl-phenylacetaldehyd,
Hydrochlorid Fp 182 – 184°C,
$[\alpha]^{20}_{58}$ g = -7,2° (c = 20.1 mg/ml, Ethanol, d = 10 cm)

Analyse:
ber.: C 73,9 H 8,6 Cl 9,5 N 3,8
gef.: C 73,8 H 8,6 Cl 9,5 N 3,8

Beispiel 3

(R)-2-[3-[(Phenethyl)methylamino]propyl]-2-isopropyl-phenylacetaldehyd,
Hydrochlorid Fp. 188 - 184°C, $[\alpha]^{20}_{58}$ g = +7,7° (c = 20.1 mg/ml, EtOH, d = 10 cm)

Analyse:
ber.: C 73,9 H 8,6 Cl 9,5 N 3,8
gef.: C 73,9 H 8,5 Cl 9,5 N 3,8

(Die optisch aktiven Ausgangsprodukte für die Synthese der Substanzen 2 und 3 sind in DE-OS 33 44 755 beschrieben.)

Beispiel 4

2-[3-[(Phenethyl)methylamino]propyl]-diphenylacetaldehyd

Analyse:
ber.: C 84,1 H 7,9 N 3,8
gef.: C 83,9 H 7,8 N 3,8

Beispiel 5

[3-[(3-Methoxyphenethyl)methylamino]propyl]-2-dodecyl-3-methoxyphenylacetaldehyd

Analyse:
ber.: C 78,0 H 10,2 N 2,7
gef.: C 78,0 H 9,8 N 2,8

Beispiel 6

2-[3-[(3-Methoxyphenethyl)methylamino]propyl]n-2-isopropyl-3,4-dimethoxyphenylacetaldehyd

Analyse:
ber.: C 73,0 H 8,7 N 3,3
gef.: C 73,1 H 8,8 N 3,4

Beispiel 7

2-[3-[(3-Methoxyphenethyl)methylamino]propyl]-2-isopropyl-3-methoxyphenylacetaldehyd
Analyse:
ber.: C 75,5 H 8,9 N 3,5
gef.: C 75,4 H 9,0 N 3,5

Beispiel 8

2-[3-[(3,4-Dimethoxyphenethyl)methylamino]propyl]-2-isopropyl-3,4-dimethoxyphenylacetaldehyd
Ein Gemisch von 29,9 g (0,1 Mol) α-Isopropyl-α-(3-chlorpropyl)-3,4-dimethoxyphenylacetaldehyd und 19,5 g (0,1 Mol) N-Methyl-3,4-dimethoxyphenethylamin in 100 ml Acetonitril wurde in Gegenwart von 27,8 g wasserfreiem Kaliumcarbonat unter gutem Rühren 8 h unter Rückfluß erhitzt. Nach Erkalten wurde das Reaktionsgemisch in Wasser gegossen und zweimal mit je 100 ml Ether extrahiert. Nach Abziehen des Ethers wurde der ölige Rückstand säulenchromatographisch gereinigt (Kieselgel, Elutionsmittel: Methylenchlorid/Ethanol, 9/1). Die isolierte Base wurde in 300 ml heißen Isopropanol gelöst und mit isopropanolischer Oxalsäurelösung versetzt.
Es wurden 36,7 g (67 %) Hydrogenoxalat vom Schmelzpunkt 138 - 142°C (Zersetzung) isoliert.
Analog Beispiel 8 wurden erhalten:

Beispiel 9

2-[3-[(3-Methoxyphenethyl)methylamino]propyl]-2-isopropyl-3,4,5-trimethoxyphenylacetaldehyd, Hydrogenoxalat: Fp 118 - 120°C.

Beispiel 10

2-[3-[(3,4-Dimethoxyphenethyl)methylamino]propyl]-2-isopropyl-3,4,5-trimethoxyphenylacetaldehyd, Hydrogenoxalat: Fp 143- 145°C.

Beispiel 11

2-[3-[(3,5-Dimethoxyphenethyl)methylamino]propyl]-2-isopropyl-3,5-dimethoxyphenylacetaldehyd, Hydrogenoxalat: Fp 97 - 100°C.

Beispiel 12

2-[3-[(3,5-Diethoxyphenethyl)methylamino]propyl]-2-isopropyl-3,5-diethoxyphenylacetaldehyd, Hydrogenoxalat: Fp 115 - 117°C.
Analog Beispiel 1 und 2 lassen sich darstellen:

Beispiel 13

2-[3-[(Phenethyl)methylamino]propyl]-2-isopropyl-3,4-dichlorphenylacetaldehyd,

Beispiel 14

2-[3-[(3,4-Dimethoxyphenethyl)methylamino]propyl]-2-isopropyl-4-nitrophenylacetaldehyd,

Beispiel 15

2-[3-[(3-Nitrophenethyl)methylamino]propyl]-2-isopropyl-3,4,5-trimethoxyphenylacetaldehyd,

Beispiel 16

2-[3-[(3,4-Dimethylphenethyl)methylamino]propyl]-2-isopropyl-3,5-dimethoxyphenylacetaldehyd,

Beispiel 17

2-[3-[(3,4-Dichlorphenethyl)methylamino]propyl]-2-isopropyl-3,4-dimethoxyphenylacetaldehyd,

Beispiel 18

2-[3-[(4-Fluorphenethyl)methylamino]propyl]-2-isopropyl-3,5-dimethoxyphenylacetaldehyd,

Beispiel 19

2-[3-[(3,5-Dimethoxyphenethyl)methylamino]propyl]-2-isopropyl-3,4-dichlorphenylacetaldehyd,

Beispiel 20

2-[3-[(3,5-Diethoxyphenethyl)methylamino]propyl]-2-isopropyl-3,5-dimethoxyphenylacetaldehyd,

Beispiel 21

2-[3-[(4-Chlorphenethyl)methylamino]propyl]-2-isopropyl-3,5-dimethoxyphenylacetaldehyd,

Beispiel 22

2-[3-[(3-Trifluormethylphenethyl)methylamino]propyl]-2-isopropyl-3,5-dimethoxyphenylacetaldehyd,

Beispiel 23

2-[3-[(3,4-Dimethoxyphenethyl)methylamino]propyl]-2-isopropyl-3-trifluormethylphenylacetaldehyd,

Beispiel 24

2-[3-[(3,5-Diethoxyphenethyl)methylamino]propyl]-2-isopropyl-phenylacetaldehyd,

Beispiel 25

2-[3-[(3,4-Diethoxyphenethyl)methylamino]propyl]-2-isopropyl-3,4,5-trimethoxyphenylacetaldehyd,

Beispiel 26

2-[3-[(3,4-Dimethoxyphenethyl)methylamino]propyl]-2-isopropyl-4-butylphenylacetaldehyd,

Beispiel 27

2-[3-[(3-t-Butoxyphenethyl)methylamino]propyl]-2-isopropyl-3-t-butoxyphenylacetaldehyd,

Beispiel 28

2-[3-[(3,3-Methoylendioxyphenethyl)methylamino]propyl]-2-isopropyl-3,5-dimethoxyphenylacetaldehyd,

Beispiel 29

2-[3-[(3,4-Dimethoxyphenethyl)methylamino]propyl]-2-n-octyl-3,4,5-trimethoxyphenylacetaldehyd,

Beispiel 30

2-[3-[(3-Methoxyphenethyl)methylamino]propyl]-2-isopropyl-1,2,3,4-tetrahydronaphthalin-6-acetaldehyd,

Beispiel 31

2-[3-[(Phenethyl)methylamino]propyl]-2-isopropyl-indanyl-5-acetaldehyd,

Beispiel 32

2-[3-[(1,4-Benzodioxamyl-6-ethyl)methylamino]propyl]-2-isopropyl-3,4,5-trimethoxyphenylacetaldehyd,

Beispiel 33

2-[3-[(3,5-Dimethoxyphenethyl)methylamino]propyl]-2-isopropyl-3,4-ethylendioxyphenylacetaldehyd,

Beispiel 34

2-[3-[(1,3-Benzodioxanyl-6-ethyl)methylamino]propyl]-2-isopropyl-3,4,5-trimethoxyphenylacetaldehyd,

Beispiel 35

2-[3-[(Phenylpropyl)methylamino]propyl]-2-isopropyl-3,4,5-trimethoxyphenylacetaldehyd,

Beispiel 36

2-[3-[(Phenethyl)methylamino]propyl]-2-cyclohexyl-phenylacetaldehyd,

Beispiel 37

2-[3-[(3-Methoxyphenethyl)methylamino]propyl]-2-isopropyl-3,4,5-trimethoxyphenylacetaldehyd,

Beispiel 38

2-[4-[(3-Methoxyphenethyl)methylamino]butyl]-2-isopropyl-phenylacetaldehyd.

Beispiel A

Auf einer Tablettenpresse werden in üblicher Weise Tabletten folgender Zusammensetzung gepreßt:
40 mg Substanz des Beispiels 1
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6 %iger Kleister)

Beispiel B

In üblicher Weise werden Dragees folgender Zusammensetzung hergestellt:
20 mg Substanz des Beispiels 1
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel C

10 g Substanz des Beispiels 1 werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche

1. Basisch substituierte Phenylacetaldehyde der Formel I

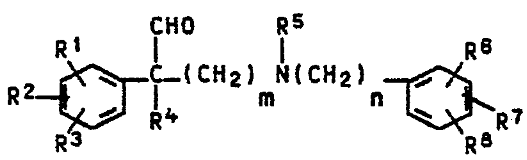

I

worin

$R^1$, $R^2$, $R^3$, $R^6$, $R^7$, $R^8$ gleich oder verschieden sind und Wasserstoffatome, Halogenatome, Trifluormethylgruppen, $C_1$-$C_4$-Alkylgruppen, Nitrogruppen oder $C_1$-$C_4$-Alkoxygruppen bedeuten, wobei auch jeweils zwei Reste in Nachbarstellung zusammen Methylendioxy-, Ethylendioxy-, 1,3-Dioxatetramethylen-, Propylen- oder Butylengruppen bilden können, $R^4$ eine gesättigte oder ungesättigte $C_1$-$C_{12}$-Alkylgruppe, eine Cycloalkyl- oder Arylgruppe ist,
$R^5$ einen $C_1$-$C_4$-Alkylrest darstellt und
m und n gleich oder verschieden sind und die Zahlen 2 bis 4 bedeuten,
sowie deren Salze mit physiologisch verträglichen Säuren.

2. (RS)-2-[3-[(Phenethyl)methylamino]propyl]-2-isopropyl-phenylacetaldehyd und seine Enantiomeren,

3. (RS)-2-[3-[(3-Methoxyphenethyl)methylamino]propyl]-2-isopropyl-3,4-dimethoxyphenylacetaldehyd und seine Enantiomeren,

4. (RS)-2-[3-[(3-Methoxyphenethyl)methylamino]propyl]-2-isopropyl-3,4,5-trimethoxyphenylacetaldehyd und seine Enantiomeren,

5. (RS)-2-[3-[(3,5-Dimethoxyphenethyl)methylamino]propyl]-2-isopropyl-3,5-dimethoxyphenylacetaldehyd und seine Enantiomeren,

6. (RS)-2-[3-[(3-Ethoxyphenethyl)methylamino]propyl]-2-isopropyl-3-ethoxyphenylacetaldehyd und seine Enantiomeren,

7. (RS)-2-[3-[(3,5-Diethoxyphenethyl)methylamino]propyl]-2-isopropyl-3,5-diethoxyphenylacetaldehyd und seine Enantiomeren.

8. Verfahren zur Herstellung der basisch substituierten Phenylacetaldehyde der Formel I gemäß Anspruch 1, dadurch gekennzeichnet, daß man

a) Phenylacetonitrile der Formel II

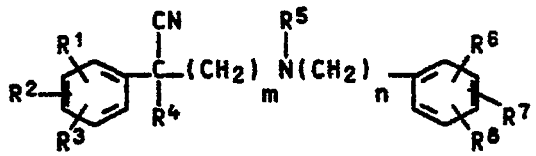

II,

worin $R^1$ bis $R^8$, m und n wie oben angegeben definiert sind, mit einem komplexen Aluminiumhydrid umsetzt und anschließend hydrolisiert oder

b) Phenylacetaldehyde der Formel III

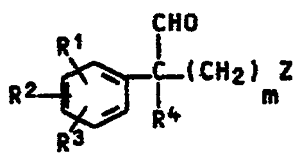

III,

worin $R^1$ bis $R^4$ und m die oben angegebene Bedeutung haben und Z eine Austrittsgruppe darstellt, mit einem Phenylalkylamin der Formel IV

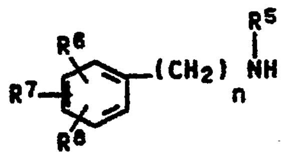

IV,

worin $R^5$ bis $R^8$ und n die obige Bedeutung besitzen, zur Reaktion bringt und die so erhaltene Verbindung gewünschtenfalls in ein Salz mit einer physiologisch verträglichen Säure überführt.

9. Verbindungen der Formel I gemäß Anspruch 1 zur Verwendung bei der Bekämpfung von Krankheiten.

## Claims

1. A phenylacetaldehyde substituted by basic groups, of the formula I

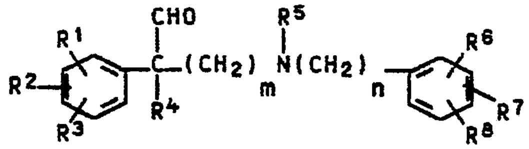

I

where $R^1$, $R^2$, $R^3$, $R^6$, $R^7$ and $R^8$ are identical or different and are each hydrogen, halogen, trifluoromethyl, $C_1$–$C_4$-alkyl, nitro or $C_1$–$C_4$-alkoxy, and two radicals in adjacent positions may furthemore together form methylenedioxy, ethylenedioxy, 1,3-dioxatetramethylene, propylene or butylene, $R^4$ is a saturated or unsaturated $C_1$–$C_{12}$-alkyl group, a cycloalkyl group or an aryl group, $R^5$ is $C_1$–$C_4$-alkyl and m and n are identical or different and are each 2, 3 or 4, and its salts with physiologically tolerated acids.

2. (RS)-2-[3[(Phenylethyl)-methylamino]-propyl]-2-isopropylphenylacetaldehyde and its enantiomers.

3. (RS)-2-[3-[(3-Methoxyphenylethyl)-methylamino]-propyl]-2-isopropyl-3,4-dimethoxyphenylacetaldehyde and its enantiomers.

4. (RS)-2-[3-[(3-Methoxyphenylethyl)-methylamino]-propyl]-2-isopropyl-3,4,5-trimethoxyphenylacetaldehyde and its enantiomers.

5. (RS)-2-[3-[(3,5-Dimethoxyphenylethyl)-methylamino]-propyl]-2-isopropyl-3,5-dimethoxyphenylacetaldehyde and its enantiomers.

6. (RS)-2-[3-[(3-Ethoxyphenylethyl)-methylamino]-propyl]-2-isopropyl-3-ethoxyphenylacetaldehyde and its enantiomers.

7. (RS)-2-[3-[(3,5-Diethoxyphenylethyl)-methylamino]-propyl]-2-isopropyl-3,5-diethoxyphenylacetaldehyde and its enantiomers.

8. A process for the preparation of a phenylacetaldehyde which is substituted by basic groups, of the formula I according to claim 1, wherein

a) a phenylacetonitrile of the formula II

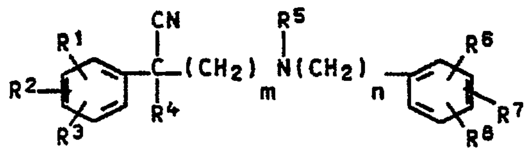

II

where $R^1$ to $R^8$, m and n are as defined above, is reacted with a complex aluminum hydride and then hydrolyzed, or

b) a phenylacetaldehyde of the formula III

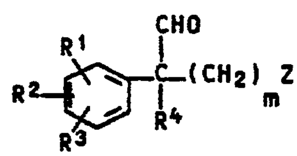

III

where $R^1$ to $R^4$ and m have the above meanings and Z is a leaving group, is reacted with a phenylalkylamine of the formula IV

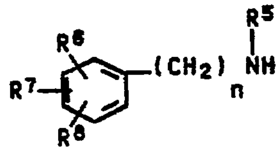

IV

where $R^5$ to $R^8$ and n have the above meanings, and, if desired, the resulting compound is converted to a salt with a physiologically tolerated acid.

9. A compound of the formula I as claimed in claim 1 for use in the treatment of disorders.

## Revendications

1. Phénylacétaldéhydes substitués basiquement, de formule I

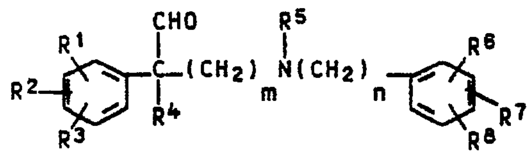

I

dans laquelle
$R^1$, $R^2$, $R^3$, $R^6$, $R^7$, $R^8$ sont identiques ou différents et représentent des atomes d'hydrogène, des atomes d'halogène, des groupes trifluorométhyle, des groupes alkyle en $C_1$–$C_4$, des groupes nitro ou des groupes alcoxy en $C_1$–$C_4$, deux restes en positions voisines pouvant chaque fois former aussi ensemble des groupes méthylènedioxy, éthylènedioxy, 1,3-dioxatétraméthylène, propylène ou butylène,
$R^4$ est un groupe alkyle en $C_1$–$C_{12}$ saturé ou non saturé, un groupe cyclo-alkyle ou -aryle
$R^5$ représente un reste alkyle en $C_1$–$C_4$ et m et n sont égaux ou différents et représentent les nombres 2 à 4,
ainsi que leurs sels d'acides tolérables physiologiquement.

2. (RS)-2-[3-[(phénéthyl)méthylamino]propyl]-2-isopropylphénylacétaldéhyde et ses énantomères.

3. (RS)-2-[3-[(3-méthoxyphénythyl)méthylamino]propyl]-2-isopropyl-3,4-diméthoxyphénylacétaldehyde et ses énantiomères.

4. (RS)-2-[3-[(3-méthoxyphénéthyl)méthylamino]propyl]-2-isopropyl-3,4,5-triméthoxyphénylacétaldéhyde et ses énantiomères.

5. (RS)-2-[3-[(3,5-diméthoxyphénéthyl)méthylamino]propyl]-2-isopropyl-3,5-diméthoxyphénylacétaldéhyde et ses énantiomères.

6. (RS)-2-[3-[(3-éthoxyphénéthyl)méthylamino]propyl]-2-isopropyl-3-éthoxyphénylacétaldéhyde et ses énantiomères.

7. (RS)-2-[3-[(3,5-diéthoxyphénéthyl)méthylamino]propyl-2-isopropyl-3,5-diéthoxyphénylacétaldéhyde et ses énantiomères.

8. Procédé de préparation des phénylacétaldéhydes substitués basiquement de formule I selon la revendication 1, caractérisé par le fait qu'on fait réagir:

a) des phénylacétonitriles de formule II

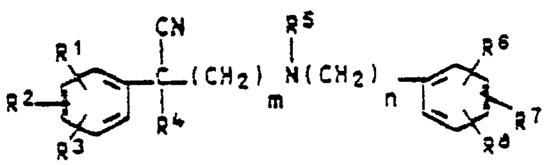

II,

où $R^1$ à $R^8$, m et n sont définis comme indiqué plus haut, avec un hydrure d'aluminium complexe et/on hydrolyse ensuite, ou bien

b) des phénylacétaldéhydes de formule III

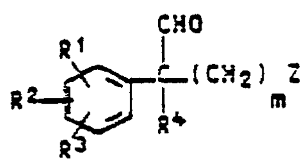

III,

où $R^1$ à $R^4$ et m ont la signification donnée plus haut et Z représente un groupe éliminable, avec une phénylalkylamine de formule IV

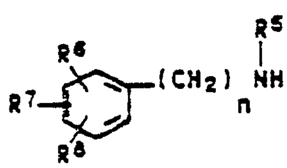

IV,

où $R^5$ à $R^8$ et n ont la signification ci-dessus et, éventuellement, on transforme le composé ainsi obtenu en un sel d'un acide tolérable physiologiquement.

9. Composés de formule I selon la revendication 1 pour utilisation dans la lutte contre les maladies.